**Europäisches Patentamt**

**European Patent Office**

**Office européen des brevets**

(11) Veröffentlichungsnummer : **0 473 907 B1**

(12) **EUROPÄISCHE PATENTSCHRIFT**

(45) Veröffentlichungstag der Patentschrift :
**30.03.94 Patentblatt 94/13**

(21) Anmeldenummer : **91111557.4**

(22) Anmeldetag : **11.07.91**

(51) Int. Cl.$^5$ : **B01J 29/28,** C10G 45/64,
C07C 5/27, C07C 2/66,
C07C 1/20

(54) Verfahren zur katalytischen Umsetzung von Kohlenwasserstoffen unter Verwendung eines synthetischen kristallinen Alumosilikates.

(30) Priorität : **11.07.90 DE 4022140**

(43) Veröffentlichungstag der Anmeldung :
**11.03.92 Patentblatt 92/11**

(45) Bekanntmachung des Hinweises auf die
Patenterteilung :
**30.03.94 Patentblatt 94/13**

(84) Benannte Vertragsstaaten :
**AT BE CH DE DK ES FR GB GR IT LI LU NL SE**

(56) Entgegenhaltungen :
**EP-A- 0 138 680**
**EP-A- 0 170 751**
**EP-A- 0 406 474**
**US-A- 4 825 444**
**WORLD PATENTS INDEX LATEST Week 1790,**
**Derwent Publications Ltd., London, GB; AN**
**90-1227335 & JP-A-2 074 516**
***Zusammenfassung***

(73) Patentinhaber : **VAW Aluminium AG**
**Georg-von-Boeselager-Strasse 25**
**D-53117 Bonn (DE)**
Patentinhaber : **LEUNA-WERKE AG**
**Am Haupttor**
**D-06236 Leuna (DE)**

(72) Erfinder : **Thome, Roland Dr.**
**Brüsseler Strasse 58**
**W-5300 Bonn 1 (DE)**
Erfinder : **Tissler, Arno Dr.**
**Koernicke Strasse 2**
**W-5300 Bonn 1 (DE)**
Erfinder : **Becker, Karl Dr.**
**August-Bebel-Strasse 2**
**W-4803 Bad Kösen (DE)**
Erfinder : **Neubauer, Hans-Dieter Dr.**
**Sorbenweg 4**
**W-4300 Merseburg (DE)**
Erfinder : **John, Hans-Dieter Dr.**
**Brüderstrasse 15**
**W-4020 Halle (DE)**

(74) Vertreter : **Müller-Wolff, Thomas**
**HARWARDT NEUMANN, Patent- und**
**Rechtsanwälte, Postfach 14 55**
**D-53704 Siegburg (DE)**

**EP 0 473 907 B1**

## Beschreibung

Die vorliegende Erfindung betrifft ein Verfahren zur katalytischen Umsetzung von Kohlenwasserstoffen bzw. deren Derivaten bei Drücken im Bereich von 0,1-15 MPa, Temperaturen im Bereich 523-873 K und Katalysatorbelastungen von 0,5-8 g/h Edukt pro g Katalysator unter Verwendung eines synthetischen kristallinen Alumosilikates in geformten Katalysatoren, d.h. als Katalysatorkomponente.

Die Entwicklung und Anwendung von Molsiebkatalysatoren mit formselektiven Eigenschaften hat der Entwicklung der Erdölverarbeitung und Petrochemie ohne Zweifel in den letzten Jahrzehnten wesentliche Impulse verliehen. In besonderem Maße gilt das seit der Entdeckung der mittelporigen, siliziumreichen Zeolithe vom Typ der Pentasile. Stimuliert wurde diese Entwicklung im besonderen Maße von äußeren Faktoren. Waren es bis in die jüngste Vergangenheit hinein vor allem die drastischen Veränderungen der Erdölpreise und Erdölverfügbarkeit, die weltweit wesentliche Aktivitäten in Forschung und Entwicklung auf diesem Gebiet ausgelöst haben, so wirken heute neben den ständig wirksamen Faktoren der Intensivierung und Kostensenkung vor allem die steigenden Anforderungen des Umweltschutzes und der damit vebundenen Umweltgesetzgebung.

In der Erdölverarbeitung und Erdölchemie betrifft das vor allem

- die neuen Anforderungen an die Qualität von Vergaserkraftstoffen hinsichtlich Bleifreiheit, Senkung des Benzengehalts und vermutlich zu erwartender Senkung des Dampfdruckes,
- die Ablösung wenig umweltfreundlicher Aromaten-Alkylierungs- und Olefin-Hydratationsprozesse auf Basis konzentrierter flüssiger oder geträgerter Mineralsäuren (Schwefelsäure, Flußsäure, Phosphorsäure) als Katalysatoren,
- zu erwartende erhöhte Anforderungen an die Begrenzung des Aromaten- und Schwefelgehaltes von Dieselkraftstoffen.

Besonders für die Lösung der beiden erstgenannten Aufgaben bieten formselektive Katalysatoren auf Basis von Pentasil-Zeolithen neue Möglichkeiten. Sie schaffen zugleich und unabhängig von Forderungen des Umweltschutzes neue Möglichkeiten zur technologisch und kostenmäßig vorteilhafteren Gestaltung der Verarbeitung von Erdölfraktionen. Das trifft zu z.B. für die Prozesse der Entparaffinierung von Destillaten (Kerosin, Gasöl) und Schmierölen durch formselektives Spalten der n-Paraffine im Einsatzprodukt.

Pentasil-Zeolithe sind charakterisiert durch ein in zwei Richtungen ausgedehntes intrakristalline System sich kreuzender Kanäle mit einem Durchmesser von etwa 5,5 Å. Die Kreuzungsbereiche besitzen einen sehr schwach ausgeprägten Käfigcharakter und sind häufig der Ort des Reaktionsgeschehens. Porenform und Porengröße haben neben der Säurestärke der aciden Zentren und ihrer Konzentration entscheidenden Einfluß auf Aktivität und Selektivität der Umwandlung von Stoffen und Stoffgemischen. Zwischen der Konzentration der sauren Brönsted-Zentren und der Anzahl der Gitteraluminium-Atome pro Elementarzelle besteht ein linearer Zusammenhang.

Die Größe der Porenkanäle erlaubt den Ein- und Austritt von un- bzw. einfachverzweigten Aliphaten und von einkernigen Aromaten bis zu maximal 10 Kohlenstofatomen.

Grundsätzlich können demzufolge nur Moleküle dieser Stoffklassen innerhalb des Porengefüges chemisch umgewandelt werden und dieses wieder verlassen. Darin besteht das Wesen der sogenannten Reaktanten- und Produkt-Formselektivität. Selbst innerhalb dieser Substanzklassen resultieren aus Unterschieden in der Molekülgröße zum Teil erhebliche Unterschiede in der Geschwindigkeit der intrakristallinen Diffusion, wodurch zusätzliche Formselektivitätseffekte entstehen (z.B. Xylenisomerisierung). Reaktionen zwischen Molekülen dieser Stoffklassen, für deren Übergangszustand (aktivierter Komplex) ein größerer Raumbedarf erforderlich ist, als er von den Kreuzungsbereichen der Pentasil-Zeolithe befriedigt werden kann, verlaufen nicht oder nur mit sehr geringer Wahrscheinlich ("Übergangszustands-Formselektivität").

Damit im Zusammenhang steht die wichtigste katalytische Eigenschaft der Pentasil-Zeolithe, ihre sehr geringe Verkokungsneigung. Durch diese Eigenschaft ist die Nutzung ihrer stark aciden Zentren für die katalytische Stoffwandlung technisch lohnend, da sie zum Teil sehr lange Fahrperioden zwischen den Katalysatorregenerierungen erlaubt.

Entsprechend ihrem Charakter als anorganische, kristalline Feststoffsäuren katalysieren die Pentasil-Zeolithe in ihrer protonierten Form folgende Reaktionstypen:

- Dehydratation/Hydratation (Ether und Alkene aus Alkoholen, Alkohole aus Alkenen)
- C-C-Knüpfungsreaktionen (Oligomerisierung von Alkenen, Kondensationen sauerstoffhaltiger Verbindungen und Alkylierung von Aromaten und Isoparaffinen)
- C-C-Spaltung (Crackprozesse von Paraffinen und Alkenen)
- Aromatisierung (Aromatenherstellung aus Paraffinen und Alkenen)
- Isomerisierungen (Skelett- und Doppelbindungsisomerisierungen)

Die Synthese siliziumreicher Zeolithe der Pentasil-Familie wurde erstmals 1967 von Argauer und Landolt (US-Patent 3.702.886) beschrieben.

Die Herstellung dieser Stoffe gelang aber nur unter Zusatz von organischen, strukturlenkenden Verbindungen zum Synthesegemisch.

Meist wurden Tetraalkylammoniumverbindungen wie z.B. Tetrapropylammoniumbromid hierzu verwendet. In den folgenden Jahren gelang die Synthese mit einer Vielzahl weiterer organischer Substanzen, wie sekundären Aminen, Alkoholen, Ethern, Heterocyclen und Ketonen.

Alle diese Synthesevarianten haben aber eine Reihe von schwerwiegenden Nachteilen, die eine großtechnische umweltschonende Herstellung ausschließen. Die eingesetzten Stoffe sind toxisch und leicht entflammbar. Da die Synthese unter hydrothermalen Bedingungen unter hohem Druck, meist in Autoklaven, durchgeführt werden muß, ist ein Entweichen dieser Stoffe nie ganz auszuschließen.

Hierdurch entsteht ein hohes Gefährdungspotential für die Bedienungsmannschaften und die nähere und weitere Umgebung der Produktionsstätte. Die bei der Herstellung anfallenden Abwässer enthalten ebenfalls diese Stoffe und müssen daher unter hohen Kosten entsorgt werden, um eine Umweltverseuchung auszuschließen. Hinzu kommt noch, daß die sich im Gitter befindlichen organischen Anteile bei hohen Temperaturen ausgebrannt werden müssen. Sie oder mögliche Abbau- bzw. Folgeprodukte gelangen damit in die Abluft. Dieses Ausbrennen kann zusätzlich Gitterschädigungen im Zeolith-Katalysator hervorrufen, die ihn in seinen katalytischen Eigenschaften beeinträchtigen.

Alle diese Nachteile haben dazu geführt, daß eine großtechnische Herstellung dieses nützlichen Katalysators bis heute nur in Ansätzen durchgeführt wird.

In den letzten Jahren wurden in der Patentliteratur einige Herstellungsverfahren beschrieben, bei denen auf den Einsatz dieser organischen Stoffe verzichtet werden konnte (z.B. US 4.257.885).

Die in diesen Patenten beschriebenen Herstellungsverfahren führen aber nur sehr langsam (mehrere Tage) und meist unvollständig zum gewünschten Produkt. Des weiteren ist das Auftauchen von unerwünschten Nebenphasen in der Regel nicht auszuschließen.

Mittlerweile ist bekannt, daß die Acidität und die Porenstruktur zwar notwendige Voraussetzungen aber keine hinreichenden für die Gesamtwirkung der Katalysatorkomponente und des Gesamtkatalysators sind.

Insbesondere das Langzeitverhalten dieser Katalysatoren wird weitgehend durch feine Unterschiede innerhalb der Alumosilikat struktur bestimmt. So ist z. B. bekannt, daß die Aluminiumverteilung über den Kristallquerschnitt in Pentasil-Zeolithen bei Synthesen unter Verwendung von organischen Schablonenverbindungen eine andere ist als bei Pentasil-Zeolithen, die aus rein anorganischen Syntheseansätzen erhalten werden (s. z. B. A. Tißler et al. Stud. Surf. Sci. Catal. <u>46</u> (1988) 399-408). Bei ersteren beobachtet man z. B. eine Anreicherung des Aluminiums in der Peripherie der Kristallite, bei letzteren dominiert eine Aluminiumgleichverteilung über den Kristallquerschnitt. Die Angabe eines Röntgendiffraktogramms zur Charakterisierung eines derartigen Stoffes aus der Sicht des Nutzens für die Katalyse ist somit nicht ausreichend und bedarf einer Ergänzung durch subtilere Methoden.

Aufgabe der vorliegenden Erfindung ist die Entwicklung eines Verfahrens zur katalytischen Umsetzung von Kohlenwasserstoffen, bei dem die Nachteile der bekannten Verfahren weitgehend vermieden werden unter Erzielung von Verbesserungen insbesondere hinsichtlich Umsatz, Produktausbeute, sowie Aktivität, Selektivität und Standzeiten innerhalb der Regenerierungszyklen der Katalysatoren.

Diese Aufgabe wird erfindungsgemäß gelöst durch ein Verfahren mit den Merkmalen des Anspruchs 1.

Das erfindungsgemäße Verfahren zur katalytischen Umsetzung von Kohlenwasserstoffen in petrochemischen Prozessen ist gekennzeichnet durch die Verwendung eines synthetischen Alumosilikates, das sich durch eine hohe Langzeitstabilität auszeichnet und das durch ein rein anorganisches Syntheseverfahren, das die Bildung von Nebenphasen nahezu ausschließt und das in kurzer Zeit durchgeführt werden kann, hergestellt werden kann. Diese synthetischen Alumosilikate weisen darüber hinaus physikalisch-chemische Merkmale aufweisen, die sie von ähnlichen, anders hergestellten Produkten, eindeutig unterscheidbar machen.

Diese synthetischen, kristallinen Alumosilikate weisen eine chemische Zusammensetzung auf, die in molaren Verhältnissen folgendermaßen beschrieben werden kann:

$$0\text{-}3M_2O : Al_2O_3 : 15\text{-}40SiO_2 : 0\text{-}4OH_2O,$$

wobei M ein Alkalikation bedeutet. Diese Kationen können mit Hilfe einer Mineralsäure, einer Ammoniumverbindung, anderen Protonenlieferanten oder mit anderen Kationen ausgetauscht werden.

In Verbindung mit der obenstehenden chemischen Zusammensetzung zeigen die erfindungsgemäß verwendeten Alumosilikate ein Röntgendiffraktogramm, das zumindest die in Tabelle 1 aufgelisteten Netzebenenabstände beinhaltet.

In Verbindung mit der obenstehenden chemischen Zusammensetzung und den in Tabelle 1 aufgelisteten Netzebenenabständen weisen die erfindungsgemäß verwendeten Alumosilikate eine weitgehend homogene Aluminiumverteilung über den Kristallquerschnitt auf, d. h. das $SiO_2/Al_2O_3$-Molverhältnis an der Kristallitoberfläche ist größer oder gleich dem des $SiO_2/Al_2O_3$-Verhältnisses im Zeolithinnern (siehe Fig. 4 und 5). Die Aluminiumanreicherung am Kristallitrand eines herkömmlichen Vergleichs-Alumosilikats zeigt Fig. 6.

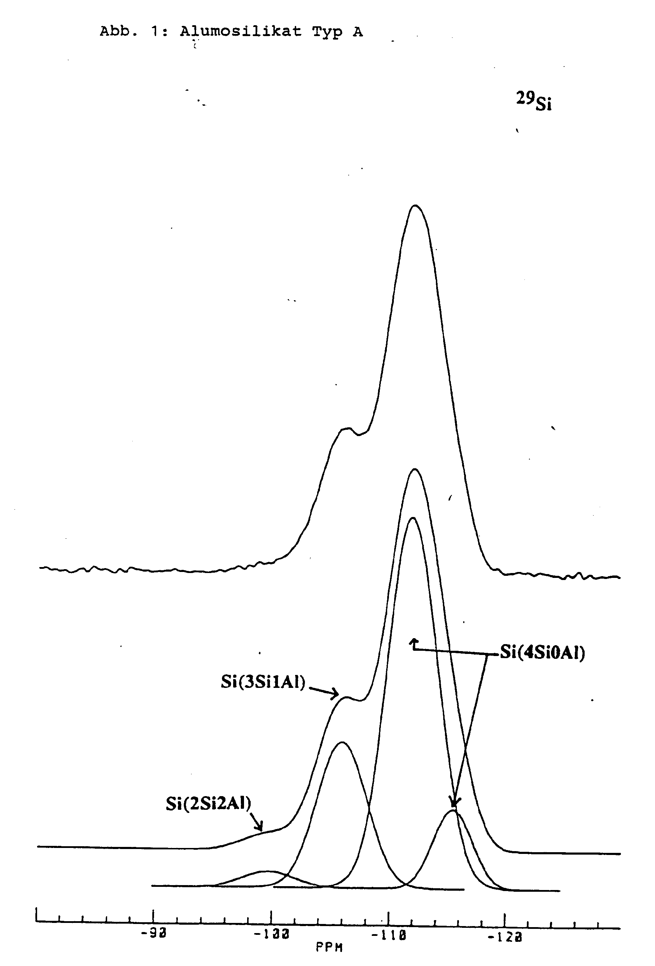

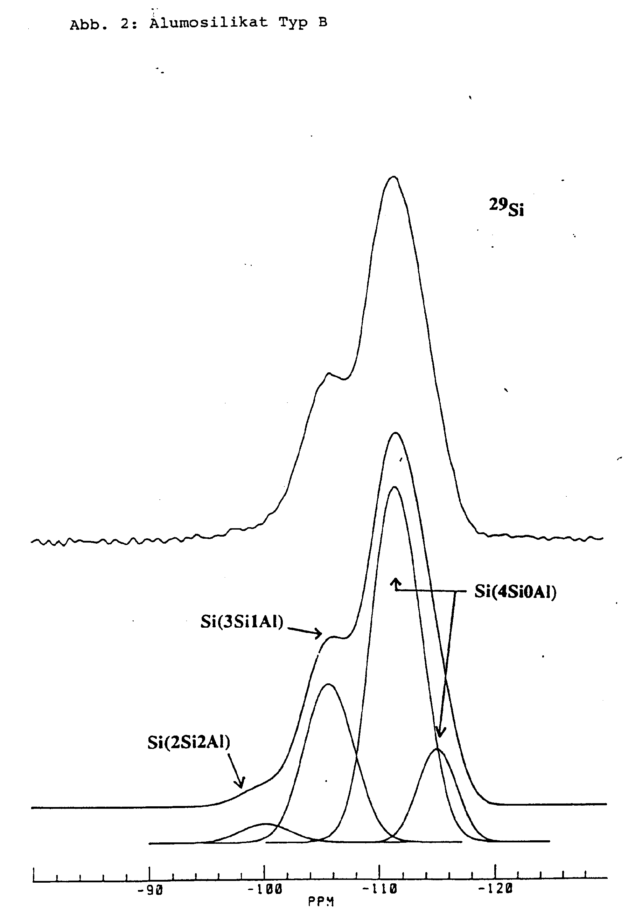

In Verbindung mit der obenstehenden chemischen Zusammensetzung und den in Tabelle 1 aufgelisteten Netzebenenabständen und der obengenannten Aluminiumgleichverteilung über den Kristallquerschnitt besitzen die erfindungsgemäß verwendeten Alumosilikate im 29-Silizium-Festkörper-MAS-Kernresonanzspektrum Adsorptionsbanden bei ca. -100, -106, -112 und -116 ppm bezogen auf Tetra-Methyl-Silan als Standard, mit dessen Hilfe diese Alumosilikate von allen ähnlichen Alumosilikaten unterschieden werden können (Fig. 1 und 2).

Das erfindungsgemäße Verfahren zur katalytischen Umwandlung von Kohlenwasserstoffen in im wesentlichen petrochemischen Prozessen basiert auf der Verwendung eines derartigen synthetischen kristallinen Alumosilikates und darauf basierender Katalysatoren , das umweltfreundlich und wirtschaftlich günstig hergestellt werden kann und das zusätzlich in vielen Fällen herkömmlichen, mit Hilfe von organischen strukturlenkenden Verbindungen hergestellten ähnlichen Alumosilikaten hinsichtlich seiner katalytischen Eigenschaften, insbesondere seiner Aktivität überlegen ist. Hierdurch kann das erfindungsgemäß verwendete synthetische, kristalline Alumosilikat in Prozessen teilweise katalysatorsparender, mit höherer Ausbeute und Selektivität eingesetzt werden.

Das erfindungsgemäß verwendete Alumosilikat zur katalytischen Umsetzung von Kohlenwasserstoffen oder deren Derivaten kann bei Drücken zwischen 0,1-15 MPa, Temperaturen im Bereich von 523-873 K und Katalysatorbelastungen von 0,5 bis 8 g/h Edukt pro g Katalysator als Katalysatorkomponente in geformten Katalysatoren in petrochemischen Verfahren eingesetzt werden und zeichnet sich in der Mehrzahl der Verfahren durch seine hohe Langzeitstabilität aus. Das erfindungsgemäß verwendete Alumosilikat kann insbesondere hergestellt werden nach dem in der EP-Anmeldung 89 115 906.3 (EP-A- 0 406 474) beschriebenen Verfahren. Das auf diese Weise hergestellte Alumosilikat wird mittels einer Ammoniumverbindung oder einer Mineralsäure ionengetauscht, ggf. durch nachfolgende Kalzination oberhalb von 573 K in die aktive Wasserstoff-Form überführt und durch Zusatz von Bindemitteln, sowie ggf. einer Metall- oder Metalloxidkomponente zu fertigen Katalysatoren umgearbeitet.

Bevorzugte Bindemittel sind amorphe Kieselsäure, Pseudoböhmit, Schichtsilikat oder eine Kombination dieser Stoffe ggf. unter Zusatz von organischen Binde- und Hilfsstoffen wie z. B. Polyvinylalkahol. Als Metall- oder Metalloxidkomponente sind Elemente der 4. bis 6. Periode des PSE, insbesondere Zn, Mo, W, Pd, Ga oder Pt bzw. eine Kombination von diesen geeignet.

Besonders vorteilhafte Eigenschaften zeigt das erfindungsgemäß verwendete Alumosilikat in Verfahren zur Entfernung von n-Paraffinen bzw. einfach verzweigten Paraffinen aus Kohlenwasserstoff-Fraktionen, in Verfahren zur Verarbeitung von $C_8$-Aromaten-Gemischen, in Verfahren zur Alkylierung von Aromaten mit niederen Alkenen, in Verfahren zur Alkylierung von Aromaten mit Alkoholen, in Verfahren zum Spalten höher siedender Kohlenwasserstoff-Fraktionen im bewegten Katalysatorbett, in Verfahren zur Isomerisierung niederer n-Paraffine zu iso-Paraffinen, in Verfahren zur Gewinnung von Aromaten aus niederen Kohlenwasserstoffen, in Verfahren zur Gewinnung von flüssigen Kohlenwasserstoffen aus niederen Alkanen bzw. Alkenen und in Verfahren zur Umsetzung von Alkoholen zu flüssigen Kohlenwasserstoffen, niederen Alkenen und zu Aromaten.

Langkettige un- bzw. geringverzweigte Paraffine haben im Vergleich zu anderen Kohlenwasserstoffen gleicher C-Zahl höhere Schmelzpunkte. Geringe Konzentrationen solcher wachsartigen Komponenten in Stoffgemischen, deren Gebrauchswert an den Paraffingehalt gebunden ist (z.B. Kraftstoffdestillate, Schmieröle), können das Fließverhalten (pour point, freeze point, cloud point) negativ beeinflussen. Anders als engporige Zeolithe (z.B. Erionit), deren formselektive Crackeigenschaften auf Moleküle des Benzinsiedebereiches beschränkt bleiben, sind mittelporige Pentasile geeignet, Paraffine mit Wachseigenschaften selektiv zu spalten und somit die entsprechenden Stoffgemische weitgehend davon zu befreien. Das Spektrum der möglichen Einsatzprodukte ist breit, es reicht vom Düsenkraftstoff bis zu Destillationsrückständen.

Eine Ausführungsform des erfindungsgemäßen Verfahrens zum Entparaffinieren von Kohlenwasserstoff-Fraktionen wird im Beispiel 4 näher beschrieben.

Einsatzprodukte für die Xylenisomerisierung enthalten alle vier der $C_8$-Aromaten, d.h. Ethylbenzen und die 3 Xylenisomeren o-, m- und p-Xylen. Eine Funktion des Isomerisierungskatalysators besteht darin, das Isomerengleichgewicht (ca. 50 % m-Xylen und je 25 % o- und p-Xylen) wieder einzustellen, nachdem ein großer Teil des p-Xylens als Produkt entfernt wurde. Darüber hinaus muß der Katalysator Ethylbenzen zu höher- oder niedrigsiedenden Nebenprodukten umwandeln, die vom Produktstrom abgetrennt werden können. Das ist notwendig, um die Anreicherung von Ethylbenzen im Kreislauf zu verhindern. Hinsichtlich der Aktivität gilt die Einschränkung, daß die Einstellung des Isomerengleichgewichts unter Bedingungen gewährleistet sein muß, die gleichzeitig eine Umwandlung des Ethylbenzens auf dem gewünschten Umsatzniveau ermöglichen. Der gewünschte Ethylbenzenumsatz ergibt sich durch die Optimierung der gesamten Isomerisierungsanlage.

Eine bevorzugte Ausführungsform des erfindungsgemäßen Verfahres zur Xylenisomerisierung wird im Beispiel 5 näher beschrieben.

Pentasil-Zeolithe haben sich zur Alkylierung von Aromaten mit niederen Alkoholen bzw. mit Olefinen bei der Gewinnung von Xylen aus Toluen und Methanol sowie von Ethylbenzen aus Benzen und Ethen (Mobil Badger Prozeß) erfolgreich als Katalysator bewährt. Darüber hinaus sind noch andere Varianten aus der Palette möglicher Alkylierungsreaktionen an Katalysatoren auf Basis von Pentasil-Zeolithen sowohl von wissenschaftlichem als auch von technischem Interesse und wurden daher in Grundlagenuntersuchungen bzw. in die angewandte Forschung einbezogen. Es handelt sich dabei vor allem um folgende Synthesen:

p-Ethyltoluen aus Toluen und Ethen,

Diethylbenzen aus Ethylbenzen und Ethen,

Dimethylethylbenzen aus Xylen und Ethen,

Cumen aus Benzen und Propen,

Alkylbenzene aus Benzen und niederen Alkoholen,

Diethylbenzen aus Ethylbenzen und Ethanol,

p-Ethyltoluen aus Toluen und Ethanol.

Eine gezielt-selektive heterogen-katalytische Gasphasenreaktion auf Pentasil-Zeolith-Basis, die zur Reife eines großtechnisch angewendeten Verfahrens entwickelt wurde, ist die Herstellung von Ethylbenzen - als Vorprodukt für Styren.

Wesentliche Vorteile gegenüber älteren Verfahren

Homogenkatalytisches $AlCl_3$-(Friedel-Craft)-Verfahren Alkar-Prozeß mit $BF_3/Al_2O_3$ als Katalysator sind die folgenden:

- keine die Umwelt belastenden Abprodukte
- keine Korrosionsprobleme
- problemlose Regenerierbarkeit des Katalysators durch Abbrennen des Kokses
- weitgehende Gewinnbarkeit der Reaktionswärme auf hohem Temperaturniveau
- Geringe Investitions- und Betriebskosten (trotz gewisser Mehraufwendungen für das Benzen-Rezyklisierungs- und das Produktreinigungssystem).

Eine Ausführungsform des erfindungsgemäßen Verfahrens zur Alkylierung von Aromaten mit niederen Alkenen wird in Beispiel 6 näher beschrieben.

Die Alkylierung von Toluen mit Methanol ist eine potentielle Alternative zum Prozeß der Xylolisomerisierung im Hinblick auf die Gewinnung von im wesentlichen para- und ortho-Xylol als Zwischenprodukte für die Kunststofferzeugung.

Eine bevorzugte Ausführungsform des erfindungsgemäßen Verfahrens zur Alkylierung von Toluol mit Methanol wird in Beispiel 7 näher beschrieben.

Katalytische Fließbett-Spalt-Verfahren nehmen eine Schlüsselrolle im Raffineriebetrieb ein. Ihre Ökonomie hängt dabei von der Effektivität des Katalysators zur Herstellung von hochwertigem Kraftstoff (RON/MON, RON = Researchoktanzahl, MON = Motoroktanzahl) oder aber LPG-Produkten (LPG = Liquefied Petroleum Gas), die für Alkylierungsreaktionen eingesetzt werden können, ab. Hauptsächlich werden die Verfahren zur Umwandlung von Vakuumdestillat eingesetzt, aber der Trend geht dahin, die Verfahren auch für den Einsatz von Destillationsrückständen zu erschließen. Auf dem Markt sind eine Vielzahl von FCC-Katalysatoren (FCC = Fluid Catalytic Cracking) im Angebot, die für unterschiedliche Benutzeranforderungen, die einmal die Ausgangsstoffe, zum anderen die gewünschten Endprodukte betreffen, maßgeschneidert sind. Heute werden vor allem Y-Zeolithe, oft ultrastabile, eingesetzt, deren Acidität durch Ionen- oder Ammoniumaustausch und anschließende thermische Behandlung erzeugt wird. Der Zeolith mit einer Teilchengröße von ca. 1 μm ist in eine Matrix eingebaut, die z.B. aus $SiO_2$-$Al_2O_3$ besteht. Der Zeolithanteil beträgt 5 - 25 Massen-%.

Seit 1983 werden Pentasil-Zeolithe zu FCC-Katalysatoren zugesetzt. Untersuchungen zeigten, daß mit dem Zusatz von 5 % SE-ausgetauschtem Pentasil zum dealuminierten SE-ausgetauschten Y-Zeolith, der hohe Kraftstoffausbeuten brachte, eine Erhöhung der Olefinausbeute von 6 auf 12 Gew.-% ($C_3$) bzw. von 9 auf 18 Gew.-% ($C_4$) erreicht würde (SE = Seltene Erden).

Der Zusatz von Pentasil-Zeolith bewirkt die Beseitigung der niedrigoktanigen Paraffinfraktion, die Bildung von $C_3$-, $C_4$-Olefinen, die Ausgangsprodukte für die Alkylierung bilden, wodurch Benzinausbeuteverluste, freie Alkylierungskapazität vorausgesetzt, zum Teil kompensiert werden können.

Ein Ausführungsbeispiel des erfindungsgemäßen Verfahrens zum Spalten höher siedender Kohlenwasserstoff-Fraktionen wird in Beispiel 8 näher beschrieben.

Eine Ausführungsform des erfindungsgemäßen Verfahrens zur Isomerisierung von niederen n-Paraffinen mit dem Ziel der Verbesserung der Frontoktanzahl von Vergaserkraftstoffen wird in Beispiel 9 näher beschrieben.

Hauptquelle aromatischer Kohlenwasserstoffe aus Erdöl ist der Reformierungsprozeß, der aber nur Kohlenwasserstoffe mit mindestens 6 Kohlenstoffatomen im Molekül in Aromaten umzuwandeln vermag. Paraffine und Olefine mit fünf und weniger Kohlenstoffatomen werden im Reformingprozeß nicht aromatisiert.

Aus den verschiedensten Gründen hat die Nutzung leichter Kohlenwasserstoffe ($C_2$-$C_5$), insbesondere der Flüssiggase, zur Gewinnung hochwertiger Flüssigprodukte in den letzten Jahren immer mehr an Bedeutung gewonnen. Die prinzipielle Möglichkeit der Dehydrocyclodimerisierung von $C_3$-$C_5$-Alkanen an Katalysatoren mit acider und dehydrierender Funktion war mindestens seit den Arbeiten von Csicsery bekannt. Der Nachteil dieser Katalysatoren lag aber in der Kürze der Arbeitsperiode zwischen den Regenerierungen. Formselektive Zeolithe, die die Koksbildung wesentlich verlangsamen, sollten hierfür besser geeignet sein.

Eine bevorzugte Ausführungsform des erfindungsgemäßen Verfahrens zur Gewinnung von Aromaten aus niederen Kohlenwasserstoffen wird in Beispiel 10 näher beschrieben.

Methanol kann an Pentasil-Zeolithen zu höheren Kohlenwasserstoffen umgesetzt werden. Je nach Katalysatormodifizierung und Verfahrensweise bei der Umsetzung können entweder ein qualitativ hochwertiges Benzin für Vergasermotoren, Aromaten für die Weiterverarbeitung z.B. in der Kunststoffindustrie oder vorzugsweise Alkene gewonnen werden. Da Methanol mit bekannten Techniken aus Erdgas oder Kohle gewonnen werden kann, ist es möglich, von diesen Rohmaterialien zu höherwertigen Kohlenwasserstoffen zu gelangen.

Eine Ausführungsform des erfindungsgemäßen Verfahrens zur Gewinnung von flüssigen Kohlenwasserstoffen oder niederen Alkenen aus Methanol wird in Beispiel 11 näher beschrieben.

**Herstellung von Katalysatoren aus den in den Beispielen 1+2+3 hergestellten Pulvern synthetischer kristalliner Alumosilikate**

Katalysator 1

Ein synthetisches kristallines Alumosilikat vom Typ B (s. Beispiel 2) wird mehrfach mit einer wässrigen Ammoniumsalzlösung (z. B. 1 N Ammoniumsulfatlösung) ionengetauscht und anschließend im Massenverhältnis 70 % Alumosilikat zu 30 % anorganischem Binder, hier Aluminiumoxid in Form von Pseudo-boemite unter Zusatz von 3 Masse% $HNO_3$, in einem Kneter vermischt und zu Extrudaten von 3 mm Durchmesser geformt und bei Temperaturen von 673 K aktiviert.

Vergleichs-Katalysator 2

Ein gemäß Beispiel 10a auf Seite 19 in "Synthesis of High-Silica Alumosilicate Zeolites" erschienen als Stud. Surf. Sci. Catal., 33 (1987), Herausgeber P.A. Jacobs und A. Martens, mit Tetrapropylammoniumbromid als strukturlenkender Verbindung synthetisierter Zeolith vom Typ C (s. Beispiel 3) wird mehrfach mit einer wässrigen Ammoniumsalzlösung ionengetauscht und anschließend im Verhältnis 70 % Alumosilikat zu 30 % anorganischem Binder, hier Aluminiumoxid in Form von Pseudo-boemite unter Zusatz von 3 Masse% $HNO_3$, in einem Kneter vermischt und zu Extrudaten von 3 mm Durchmesser geformt und bei Temperturen von 673 K aktiviert.

Katalysator 3

Ein synthetisches kristallines Alumosilikat vom Typ A (s. Beispiel 1) wird mehrfach mit einer wässrigen Ammoniumsalzlösung ionengetauscht und anschließend im Verhältnis 70 % Alumosilikat zu 30 % anorganischem Binder (s. Katalysator 1), vermischt und zu Extrudaten von 3 mm Durchmesser geformt. Anschließend wird der Katalysatorformling mit 3 Gew.% Molybdänoxid durch Tränkung mit einer ammoniakalischen Lösung von $MoO_3$ (z. B. 1 N Ammoniummolybdatlösung) belegt und bei Temperaturen von 673 K aktiviert.

Vergleichs-Katalysator 4

Ein gemäß Beispiel 10a auf Seite 19 in "Synthesis of High-Silica Alumosilicate Zeolithes" erschienen als Stud. Surf. Sci. Catal., 33 (1987), Herausgeber P.A. Jacobs und A. Martens, mit Tetrapropylammoniumbromid als strukturlenkender Verbindung synthetisierter Zeolith vom Typ C wird mehrfach mit einer wässrigen Ammoniumsalzlösung ionengetauscht und anschließend im Verhältnis 70 % Alumosilikat zu 30 % anorganischem Binder (s. Katalysator 1), vermischt und zu Extrudaten von 3 mm Durchmesser geformt. Anschließend wird der Katalysatorformling mit 3 Gew.% Molybdänoxid durch Tränkung mit einer ammoniakalischen Lösung von $MoO_3$ belegt und bei Temperaturen von 673 K aktiviert.

Katalysator 5

Ein synthetisches kristallines Alumosilikat vom Typ B wird mehrfach mit einer wässrigen Ammoniumsalzlösung ionengetauscht und anschließend im Verhältnis 70 % Alumosilikat zu 30 % anorganischem Binder (s. Katalysator 1), vermischt und zu Extrudaten von 3 mm Durchmesser geformt. Anschließend wird der Katalystor mit 2 Gew.% Galliumoxid durch Tränkung mit einer Galliumsalzlösung (z. B. Galliumchloridlösung in HCl) belegt und bei Temperaturen von 673 K aktiviert.

Vergleichs-Katalysator 6

Ein gemäß Beispiel 10a auf Seite 19 in "Synthesis of High-Silica Alumosilicate Zeolites" erschienen als Stud. Surf. Sci. Catal., 33 (1987), Herausgeber P.A. Jacobs und A. Martens, mit Tetrapropylammoniumbromid als strukturlenkender Verbindung synthetisierter Zeolith vom Typ C wird mehrfach mit einer wässrigen Ammoniumsalzlösung ionengetauscht und anschließend im Verhältnis 70 % Alumosilikat zu 30 % anorganischem Binder (s. Katalysator 1), vermischt und zu Extrudaten von 3 mm Durchmesser geformt. Anschließend wird der Katalysator mit 2 Gew.% Galliumoxid durch Tränkung mit einer Galliumsalzlösung belegt und bei Temperaturen von 673 K aktiviert.

Katalysator 7

Ein synthetisches kristallines Alumosilikat vom Typ B wird mehrfach mit einer wässrigen Ammoniumsalzlösung ionengetauscht und anschließend im Verhältnis 70 % Alumosilikat zu 30 % anorganischem Binder (s. Katalysator 1), vermischt und zu Extrudaten von 3 mm Durchmesser geformt. Anschließend wird der Katalysator mit einer Zinknitratlösung getränkt (2 Masse%) und im Wasserstoffstrom bei 40 bar $H_2$-Druck und 673 K aktiviert.

Vergleichs-Katalysator 8

Ein gemäß Beispiel 10a auf Seite 19 in "Synthesis of High-Silica Alumosilicate Zeolites" erschienen als Stud. Surf. Sci. Catal., 33 (1987), Herausgeber P.A. Jacobs und A. Martens, mit Tetrapropylammoniumbromid als strukturlenkender Verbindung synthetisierter Zeolith vom Typ C wird mehrfach mit einer wässrigen Ammoniumsalzlösung ionengetauscht und anschließend im Verhältnis 70 % Alumosilikat zu 30 % anorganischem Binder (s. Katalysator 1), vermischt und zu Extrudaten von 3 mm Durchmesser geformt. Anschließend wird der Katalysator mit einer Zinknitratlösung getränkt (2 Masse%) und im Wasserstoffstrom bei 40 bar $H_2$-Druck und 673 K aktiviert.

Beschreibung der Figuren

Fig. 1     29-Silizium-Festkörper-MAS-Kernresonanzspektrum eines Alumosilikates vom Typ A, hergestellt nach Beispiel 1.

Fig. 2     29-Silizium-Festkörper-MAS-Kernresonanzspektrum eines Alumosilikates vom Typ B, hergestellt nach Beispiel 2.

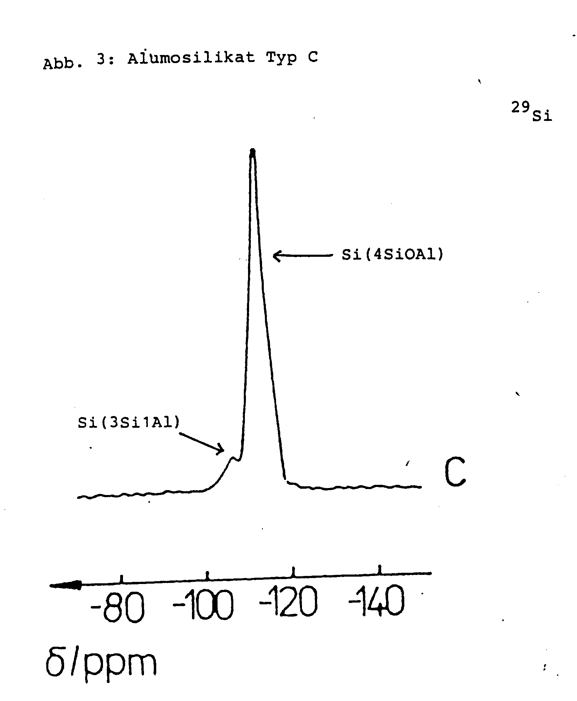

Fig. 3     29-Silizium-Festkörper-MAS-Kernresonanzspektrum eines Alumosilikates vom Typ C, hergestellt nach Beispiel 3.

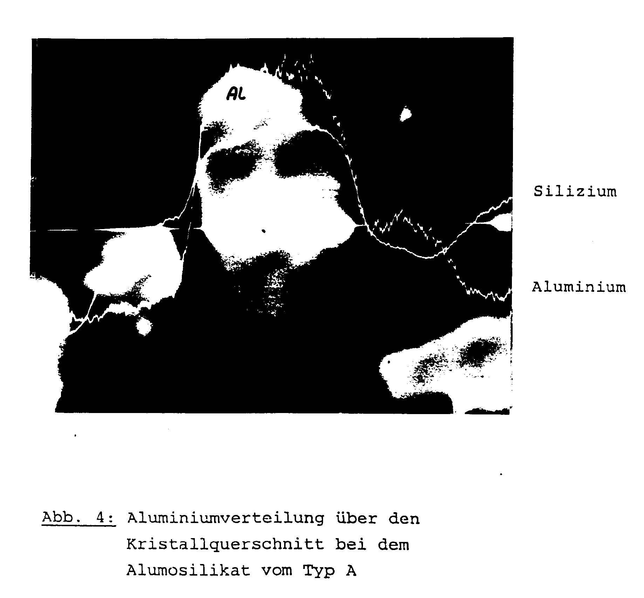

Fig. 4     Aluminium- und Siliziumverteilung über den Kristallquerschnitt eines Alumosilikates vom Typ A, hergestellt nach Beispiel 1.

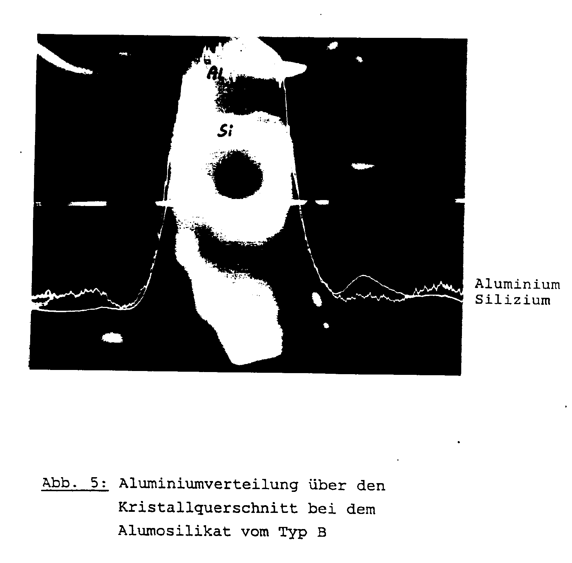

Fig. 5     Aluminium- und Siliziumverteilung über den Kristallquerschnitt eines Alumosilikates vom Typ B, hergestellt nach Beispiel 2.

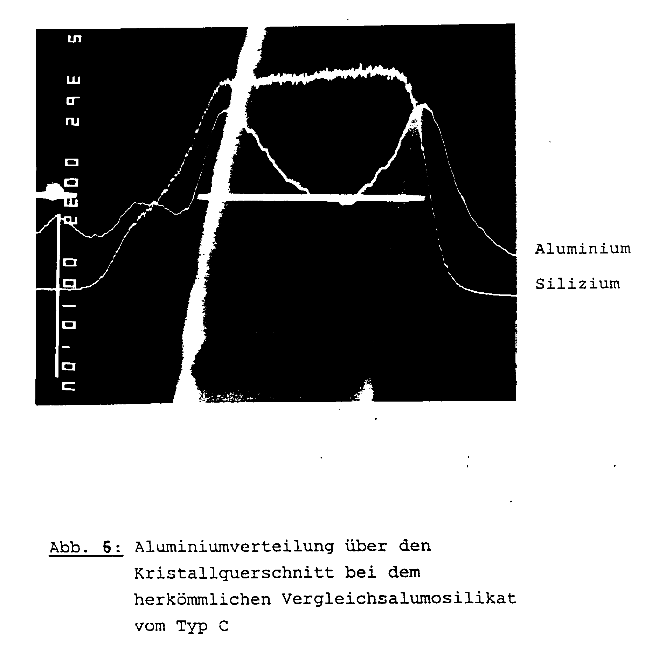

Fig. 6     Aluminium- und Siliziumverteilung über den Kristallquerschnitt eines Alumosilikates vom Typ C, hergestellt nach Beispiel 3.

Die Elementverteilungen in den Fig. 4 bis 6 wurden mit einer Elektronenstrahl-Mikrosonde IEOL IXA-733 mit angeschlossenem DEC-Rechner PDP 11/23 bestimmt. Die Proben wurden in Harz eingebettet, mit Diamantpaste abgeschliffen und mit Gold besputtert. Die Elektronenstrahl-Messungen wurden mit einer Ausgangsspannung von 15 kV und einer Stromstärke von 50 μA durchgeführt. Die Messung der Proben wurde unter Auslenken des Elektronenstrahls durchgeführt, wobei Elementverteilung und Rasterelektronenaufnahmen des Kristalls auf Photopapier aufgezeichnet wurden.

Beispiele

1. Herstellung eines Alumosilikats vom Typ A:

Ein Reaktionsansatz aus Lösungen von Na-Wasserglas, Aluminiumsulfat, Natriumsulfat und Schwefelsäure mit den Molverhältnissen

$SiO_2/Al_2O_3$ = 30
$OH^-/SiO_2$ = 0,14
$H_2O/SiO_2$ = 30

wird in einem gerührten Autoklaven auf eine Reaktionstemperatur von 185 °C aufgeheizt und 24 Stunden hydrothermal behandelt. Das feste Produkt wird filtriert und bei 110 °C getrocknet.

Die Trockensubstanz besteht aus phasenreinem Alumosilikat mit einem Röntgendiffraktogramm mit zumindest den in Tabelle 1 aufgelisteten d-Werten.

Die chemische Zusammensetzung des Produktes in molaren Verhältnissen ausgedrückt ist:

$1,1\ Na_2O : Al_2O_3 : 31 SiO_2 : 6H_2O$

Die Aluminiumverteilung über den Kristallquerschnitt des Produkts zeigt Fig. 4.

Die aus den 29-Silizium-Festkörper-MAS-Kernresonanzspektren (Fig. 1) erhaltenen Anteile der einzelnen Absorptionsbanden, die ein Maß für die verschiedenen Siliziumtetraederkoordinationen sind, liegen bei:

| Si(4SiOAl) | Si(3Si1Al) | Si(2Si2Al) |
|---|---|---|
| -112 und -116 ppm | -106 ppm | -100 ppm |
| 75 % | 23 % | 2 % |

2. Alumosilikat vom Typ B

Ein Reaktionsansatz aus Lösungen von Na-Wasserglas, Aluminiumsulfat, Natriumsulfat und Schwefelsäure mit den Molverhältnissen

$SiO_2/Al_2O_3$ = 24
$OH^-/SiO_2$ = 0,14
$H_2O/SiO_2$ = 30

wird in einem gerührten Autoklaven auf eine Reaktionstemperatur von 185 °C aufgeheizt und 24 Stunden hydrothermal behandelt. Das feste Produkt wird filtriert und bei 110 °C getrocknet.

Die Trockensubstanz besteht aus phasenreinem Alumosilikat mit einem Röntgendiffraktogramm mit zumindest den in Tabelle 1 aufgelisteten d-Werten.

Die chemische Zusammensetzung des Produktes in molaren Verhältnissen ausgedrückt ist:

$1,1\ Na_2O : Al_2O_3 : 23 SiO_2 : 7H_2O$

Die Aluminiumverteilung über den Kristallquerschnitt des Produkts zeigt Fig. 5.

Die aus den 29-Silizium-Festkörper-MAS-Kernresonanzspektren (Fig. 2) erhaltenen Anteile der einzelnen Absorptionsbanden, die ein Maß für die verschiedenen Siliziumtetraederkoordinationen sind, liegen bei:

| Si(4SiOAl) | Si(3Si1Al) | Si(2Si2Al) |
|---|---|---|
| -112 und -116 ppm | -106 ppm | -100 ppm |
| 71 % | 26 % | 3 % |

3. Herstellung eines herkömmlichen Vergleichs-Alumosilikats vom Typ C

Ein Reaktionsansatz aus pyrogener Kieselsäure, Tetrapropylammoniumbromid, Glycerol, Ammoniak, Natriumhydroxid, Aluminiumnitrat und Wasser mit den Molverhältnissen

$SiO_2/Al_2O_3$ = 72

```
Na2O/SiO2        = 0,2
TPA/SiO2         = 1,25
Glycerol/SiO2    = 19,86
NH3/SiO2         = 0,2
H2O/SiO2         = 146
```

wird in einem unbewegten Autoklaven auf eine Reaktionstemperatur von 423 K aufgeheizt und 72 Stunden hydrothermal behandelt. Das feste Produkt wird filtriert und bei 383 K getrocknet. Das Produkt besitzt ein $SiO_2/Al_2O_3$-Verhältnis von 70.

Die Aluminiumverteilung über den Kristallquerschnitt des herkömmlichen Vergleichsalumosilikat zeigt Fig. 6. Des weiteren zeigt dieses Produkt keine Absorptionsbande im 29-Silizium-Festkörper-MAS-Kernresonanzspektrum bei -100 ppm (siehe Fig. 3).

## Beispiel 4

Ein Gasöl mit einer Dichte von 0,865 kg/l, einem Stickstoffgehalt entsprechend 142 mg $NH_3$/l und einem BPA-Punkt (= Beginn der Paraffin-Ausscheidung) von 3 °C wird unter einem Druck von 1,5 MPa, bei einer Belastung von 2 g/h Edukt pro g Katalysator und einem Gasproduktverhältnis von 1000 : 1 bei 663 K Anfangstemperatur einmal an dem Katalysator 7 und an dem herkömmlichen Vergleichskatalysator 8 umgesetzt.

Die Ergebnisse der katalytischen Entparaffinierung sind in der untenstehenden Tabelle aufgelistet.

| Ergebnisse | Ausgangsprodukt | Katalysator 7 | Katalysator 8 |
|---|---|---|---|
| Dichte [kg/l] | 0,865 | 0,859 | 0,858 |
| BPA [°C] | 3 °C | -15/-25 | -15/-25 |
| Delta T/d [K] | – | 0,17 | 0,48 |

Delta T/d wurde über eine Versuchsdauer von 30 Tagen gemittelt.

Der erfindungsgemäße Katalysator 7 weist eine weit geringere fouling rate (Delta T/d) und damit eine höhere Stabilität bei etwa gleicher Anfangsaktivität auf wie der Katalysator 8.

## Beispiel 5

Ein C-8-Aromatengemisch wird bei einem Druck von 1,0 MPa, einer Temperatur von 633 K, einer Belastung von 2,0 g/h Edukt pro g Katalysator und einem Gasproduktverhältnis von 1000:1 einmal an dem Katalystor 3 und andererseits an dem herkömmlichen Vergleichskatalysator 4 umgesetzt.

Die Ergebnisse der C-8-Aromatenumsetzung sind in der untenstehenden Tabelle aufgelistet.

| Ergebnisse | Rohstoff | Katalysator 3 | Katalysator 4 |
|---|---|---|---|
| Nichtaromat. [%] | 1,09 | 0,45 | 0,71 |
| Benzen [%] | – | 14,39 | 5,32 |
| Toluen [%] | 0,71 | 6,62 | 5,04 |
| Ethylbenzen [%] | 23,75 | 2,79 | 12,24 |
| para-Xylen [%] | 9,73 | 17,89 | 16,01 |
| meta-Xylon [%] | 47,57 | 40,38 | 38,12 |
| ortho-Xylen [%] | 16,44 | 16,29 | 15,27 |
| $C9^+$ Aromaten [%] | 0,71 | 1,35 | 7,29 |
| Σ Xylene [%] | 73,74 | 74,5 | 69,4 |

Der erfindungsgemäße Katalysator 3 zeigt eine weitaus höhere Ethylbenzen-Umwandlung bei gleichzeitig besserer xylen-Selektivität (weniger $C9^+$-Aromaten) als der Vergleichskatalystor 4.

## Beispiel 6

Ein Gemisch von Benzen und Ethen im Massenverhältnis 1 : 2,6 wird bei 673 K - 693 K bei einer Belastung von 6,5 g/h Edukt (Benzen + Ethen) pro g Katalysator einmal über den Katalystor 1 und andererseits an dem herkömmlichen Vergleichskatalysator 2 umgesetzt.

Die Ergebnisse der Alkylierungsreaktion sind in der untenstehenden Tabelle aufgelistet.

| Ergebnisse | | Katalysator 1 | Katalysator 2 |
|---|---|---|---|
| Umsatz Benzol | [%] | 28 | 26 |
| Umsatz Ethen | [%] | 81 | 72 |
| Selektivität des Benzens zu Ethylbenzen + Diethylbenzen | [%] | 95 | 92 |
| Selektivität des Ethens zu Ethylbenzen + Diethylbenzen | [%] | 90 | 90 |
| Anteil para-Diethylbenzen in Diethylbenzenen | [%] | 85 | 60 |

Der erfindungsgemäße Katalysator 1 zeigt bei einer etwas höheren Aktivität eine vergleichbare Selektivität zu Ethylbenzen und Diethylbenzen, darüber hinaus aber eine deutlich verbesserte Selektivität zum para-Diethylbenzol als der Vergleichskatalysator 2.

Beispiel 7

Ein Gemisch von Toluen und Methanol im molaren verhältnis 2 : 1 wird bei 623 K bei einer Belastung von 4 g/h Edukt (Toluen + Methanol) pro g Katalysator einmal über den Katalysator 1 und andererseits an dem herkömmlichen Vergleichskatalysator 2 umgesetzt.

Die Ergebnisse der Alkylierungsreaktion sind in der untenstehenden Tabelle aufgelistet.

| Ergebnisse | | Katalysator 1 | Katalysator 2 |
|---|---|---|---|
| Methanolumsatz | [%] | 100 | 80 |
| Toluenumsatz | [%] | 30 | 17 |
| Benzen | [%] | 1 | 0,5 |
| Toluen | [%] | 51 | 61 |
| metha-Xylen | [%] | 8 | 4 |
| para-Xylen | [%] | 11 | 3,9 |
| ortho-Xylen | [%] | 7 | 3,0 |
| $\Sigma$ $C_8$-Aromaten | [%] | 26 | 11 |

Der erfindungsgemäße Katalysator 1 zeigt eine deutlich höhere Aktivität und eine leicht verbesserte para-Selektivität als der Vergleichskatalysator 2.

Beispiel 8

Der Katalysator 1 und der Katalysator 2 werden jeweils als Additive (5 Gew.%) zu einem herkömmlichen handelsüblichen Bewegtbettkatalysator auf Y-Zeolithbasis zudosiert. Nach einer Wasserdampfbehandlung bei 1023 K über 17 h der Katalysatoren wird ein hydriertes Vakuum-Destillat bei 748 K und einer Belastung von 10 g/h Edukt pro g Katalysator über die Mischkatalysatoren geleitet.

Die Ergebnisse der Crackversuche sind in der untenstehenden Tabelle aufgelistet.

| Ergebnisse | | herk. Kat. | mit % Kat. 1 | mit 5 % Kat. 2 |
|---|---|---|---|---|
| Methan | [%] | 0,5 | 0,5 | 0,5 |
| Ethan | [%] | 0,7 | 0,7 | 0,7 |
| Ethen | [%] | 0,6 | 0,5 | 0,5 |
| Propan | [%] | 3,3 | 4,7 | 3,5 |
| Propen | [%] | 2,7 | 2,9 | 2,8 |
| n-Butan | [%] | 2,0 | 1,9 | 1,9 |
| i-Butan | [%] | 6,7 | 7,9 | 6,8 |
| $\Sigma\ C_4$ | [%] | 1,5 | 1,8 | 1,7 |
| $C_5-C_{12}$ (Benzin) | [%] | 50,2 | 48,5 | 48,20 |
| Koks | | 8,4 | 5,8 | 8,1 |

Der erfindungsgemäße Katalysator bewirkt ähnlich wie der Vergleichskatalysator eine etwas höhere Gasausbeute, jedoch eine etwas bessere Benzinausbeute und einen deutlich geringeren Koksanteil. Der iso-Butan-Anteil steigt stark, was eine deutliche Verbesserung der Motoroktanzahl (MON) bewirkt. Ebenfalls erhöht sind die Anteile der $C_3$ und $C_4$-Olefine, die eine Verbesserung der Researchoktanzahl (RON) mit sich bringt.

Beispiel 9

Ein n-Hexan/Wasserstoff-Gemisch im Massenverhältnis 1:10 wird bei 573 K und 4 MPa und einer Belastung von 1 g/h Edukt pro g Katalysator einmal an dem Katalysator 7 und zum Vergleich an dem herkömmlichen Vergleichskatalysator 8 umgesetzt.

Die Ergebnisse der Reaktion sind in der untenstehenden Tabelle aufgelistet.

| Ergebnisse | Katalysator 7 | Katalysator 8 |
|---|---|---|
| n-Hexan-Umsatz (%) | 48 | 25 |
| Hexanisomere/Crackprod. | 1,3 | 1,2 |

Der erfindungsgemäße Katalysator 7 zeigt eine deutlich höhere Aktivität und ein leicht besseres Hexanisomer/Crackprodukt-Verhältnis als der herkömmliche Vergleichskatalysator.

Beispiel 10

N-Pentan wird bei einem Druck von 0,1 MPa und einer Belastung von 1 g/h Edukt pro g Katalysator bei einer Temperatur von 773 K einmal an dem Katalysator 5 und dem herkömmlichen Vergleichskatalysator 6 umgesetzt.

Die Ergebnisse der Aromatisierungsreaktion sind in der untenstehenden Tabelle aufgelistet:

| Ergebnisse | | Katalysator 5 | Katalysator 6 |
|---|---|---|---|
| Flüssigprodukt | (%) | 45 | 39,2 |
| Gase | (%) | 54,1 | 60,3 |
| Kohlenstoff | (%) | 0,9 | 0,5 |
| Aromaten | (%) | 44,5 | 38,5 |

Der erfindungsgemäße Katalysator zeigt im Vergleich zum herkömmlichen Katalysator eine verbesserte Aromatenausbeute.

Beispiel 11

Methanol wird bei einem Druck von 0,1 MPa, einer Temperatur von 573 K und einer Belastung von 1 g/h Edukt pro g Katalysator einmal an dem Katalysator 1 und an dem herkömmlichen Vergleichskatalysator 2 umgesetzt.

Die Ergebnisse der Reaktion sind in der untenstehenden Tabelle aufgelistet:

| Ergebnisse | Katalysator 1 | Katalysator 2 |
|---|---|---|
| Umsatz | 99 % | 70 % |
| Olefine | 33 % | 23 % |
| Aromaten | 12 % | 15 % |

Der erfindungsgemäße Katalysator 1 zeigt eine deutliche höhere Aktivität und eine verbesserte Olefinausbeute als der herkömmliche Vergleichskatalysator.

## Tabelle 1

| d-Werte/Netzebenenabstände [Å] | Relative Intensität |
|---|---|
| 11,2 ± 0,3 | stark |
| 10,2 ± 0,3 | stark |
| 9,8 ± 0,2 | schwach |
| 3,85 ± 0,1 | sehr stark |
| 3,83 ± 0,1 | stark |
| 3,75 ± 0,1 | stark |
| 3,73 ± 0,1 | stark |
| 3,60 ± 0,1 | schwach |
| 3,06 ± 0,05 | schwach |
| 3,00 ± 0,05 | schwach |
| 2,01 ± 0,02 | schwach |
| 1,99 ± 0,02 | schwach |

**Patentansprüche**

1.  Verfahren zur katalytischen Umsetzung von Kohlenwasserstoffen und deren Derivaten in petrochemischen Prozessen unter Verwendung von synthetischen, kristallinen

Alumosilikaten in geformten Katalysatoren,
dadurch gekennzeichnet,
daß die katalytische Umsetzung bei Drücken im Bereich von 0,1-15 MPa, bei Temperaturen im Bereich von 523 K bis 873 K und bei Katalysatorbelastungen von 0,5 bis 10 g/h Edukt pro g Katalysator durchgeführt wird, daß beim katalytischen Umsetzen

- zum Spalten höhersiedender Kohlenwasserstoff-Fraktionen in einem bewegten Katalysatorbett,
- zur Entfernung von n-Paraffinen und einfach verzweigten Paraffinen aus Kohlenwasserstoff-Fraktionen
- von $C_8$-Aromaten-Gemischen zur Gewinnung von ortho- und/oder para-Xylen
- zur Alkylierung von Aromaten mit niederen Alkenen
- zur Alkylierung von Aromaten mit Alkoholen
- zur Isomerisierung niederer n-Paraffinen zu iso-Paraffinen
- zur Gewinnung von Aromaten aus niederen Kohlenwasserstoffen

und

- von Methanol zur Gewinnung von flüssigen Kohlenwasserstoffen oder niederen Alkanen und Alkenen

als Katalysator oder als Katalysatorkomponente ein synthetisches, kristallines Alumosilikat mit der chemischen Zusammensetzung
0-3 $M_2O$ : $Al_2O_3$: 15-40 $SiO_2$ : 0-40 $H_2O$,
wobei M ein Metall-Kation bedeutet, verwendet wird, das
- zumindest die zu den d-Werten (A) gehörenden Röntgenreflexe

| 11,2 | +/- 0,3 | stark |
| 10,2 | +/- 0,3 | stark |
| 9,8 | +/- 0,2 | schwach |
| 3,85 | +/- 0,1 | sehr stark |
| 3,83 | +/- 0,1 | stark |
| 3,75 | +/- 0,1 | stark |
| 3,73 | +/- 0,1 | stark |
| 3,60 | +/- 0,1 | schwach |
| 3,06 | +/- 0,05 | schwach |
| 3,00 | +/- 0,05 | schwach |
| 2,01 | +/- 0,02 | schwach |
| 1,99 | +/- 0,02 | schwach, |

- ein größeres oder gleiches Si/Al-Molzahlenverhältnis an der Kristallitoberfläche im Vergleich zum Kristallitinneren,

und

- Absorptionsbanden des 29-Silizium-Festkörper-MAS-Kernresonanzspektrum bezogen auf den Standard Tetramethyl-silan bei ca. -100, -106, -112 und -116 ppm

aufweist.

2.   Verfahren nach Anspruch 1,
dadurch gekennzeichnet,
daß das katalytische Spalten höhersiedender Kohlenwasserstoffe wie Vakuumdestillate und hydrierte Gasöle, bei einer Temperatur zwischen 723 K und 773 K und einer Katalysatorbelastung von 10 g/h Edukt pro g Katalysator durchgeführt wird.

3.   Verfahren nach Anspruch 1,
dadurch gekennzeichnet,
daß die katalytische Umsetzung zur Entfernung von n-Paraffinen und einfach verzweigten Paraffinen aus Kohlenwasserstoff-Fraktionen bei Drücken im Bereich 1,0 bis 1,5 MPa und Temperaturen von 523 K und 723 K durchgeführt wird.

4. Verfahren nach den Ansprüche 1 und 3,
dadurch gekennzeichnet,
daß ein Gasöl bei einer Temperatur zwischen 663 K und 723 K und einer Katalysatorbelastung von 2 g/h Gasöl pro g Katalysator entparaffiniert wird.

5. Verfahren nach Anspruch 1,
dadurch gekennzeichnet,
daß die katalytische Umsetzung von $C_8$-Aromaten-Gemischen zur Gewinnung von ortho- und/oder para-Xylen bei Drücken im Bereich von 0,5 bis 5,0 MPa und Temperaturen im Bereich von 523 K bis 773 K durchgeführt wird.

6. Verfahren nach den Ansprüchen 1 und 5,
dadurch gekennzeichnet,
daß zur Isomerisierung von Xylen ein $C_8$-Aromatengemisch bei einer Temperatur zwischen 623 K und 723 K bei einer Katalysatorbelastung von 2 g/h Edukt pro Katalysator katalytisch umgesetzt wird.

7. Verfahren nach Anspruch 1,
dadurch gekennzeichnet,
daß die katalytische Umsetzung zur Alkylierung von Aromaten mit niederen Alkenen bei Drücken im Bereich von 1,0 bis 5,0 MPa und Temperaturen von 623 K bis 773 K durchgeführt wird.

8. Verfahren nach den Ansprüchen 1 und 7,
dadurch gekennzeichnet,
daß Benzen mit Ethen zu Ethylbenzen umgesetzt wird.

9. Verfahren nach den Ansprüchen 1, 7 und 8,
dadurch gekennzeichnet,
daß eine Mischung aus Benzen und Ethen in einem Massen-Verhältnis Benzen : Ethen = 1 : 2,6 bei einer Temperatur zwischen 673 K bis 693 K und einer Katalysatorbelastung von 6,5 g/h Edukt pro Katalysator katalytisch zu Ethylbenzen umgesetzt wird.

10. Verfahren nach den Ansprüchen 1 und 7,
dadurch gekennzeichnet,
daß Benzen mit Propen zu Cumen umgesetzt wird.

11. Verfahren nach Anspruch 1,
dadurch gekennzeichnet,
daß die katalytische Umsetzung zur Alkylierung von Aromaten mit Alkoholen bei Drücken im Bereich von 0,1 bis 0,5 MPa und Temperaturen im Bereich von 523 K bis 773 K durchgeführt wird.

12. Verfahren nach den Ansprüchen 1 und 11,
dadurch gekennzeichnet,
daß Toluen mit Methanol zu Xylen umgesetzt wird.

13. Verfahren nach den Ansprüchen 1, 11 und 12,
dadurch gekennzeichnet,
daß zur Alkylierung von Toluen mit Methanol eine Mischung in einem molaren Verhältnis Toluen : Methanol = 2 : 1 bei einer Temperatur zwischen 573 K und 673 K und einer Katalysatorbelastung von 4 g/h Edukt pro Katalysator umgesetzt wird.

14. Verfahren nach Anspruch 1,
dadurch gekennzeichnet,
daß die katalytische Umsetzung zur Isomerisierung niederer n-Paraffinen zu iso-Paraffinen bei Drücken im Bereich von 0,5 bis 5 MPa und Temperaturen im Bereich von 473 K bis 773 K durchgeführt wird.

15. Verfahren nach den Ansprüche 1 und 14,
dadurch gekennzeichnet,

daß die n-Paraffine bei einer Temperatur zwischen 523 K und 623 K und einer Katalysatorbelastungen von 1 g/h Edukt pro g Katalysator isomerisiert werden.

16. Verfahren nach Anspruch 1,
dadurch gekennzeichnet,
daß die katalytische Umsetzung zur Gewinnung von Aromaten aus niederen Kohlenwasserstoffen bei Drücken von 0,1 bis 5,0 MPa und Temperaturen im Bereich von 773 K bis 873 K durchgeführt wird.

17. Verfahren nach den Ansprüchen 1 und 16,
dadurch gekennzeichnet,
daß zur Herstellung von Aromaten Kohlenwasserstoffe mit niedrigem Molekulargewicht bei einer Temperatur zwischen 773 K und 873 K und einer Katalysatorbelastung von 1 g/h Edukt pro g Katalysator katalytisch umgesetzt werden.

18. Verfahren nach Anspruch 1,
dadurch gekennzeichnet,
daß die katalytische Umsetzung von Methanol zur Gewinnung von flüssigen Kohlenwasserstoffen oder niederen Alkanen und Alkenen bei Drücken im Bereich von 0,1 bis 5,0 MPa und Temperaturen im Bereich von 523 K bis 823 K durchgeführt wird.

19. Verfahren nach den Ansprüchen 1 und 18,
dadurch gekennzeichnet,
daß das Methanol katalytisch umgesetzt wird zu flüssigen Kohlenwasserstoffen von $C_4$ bis $C_{12}$ oder niederen Alkenen $< C_6$, bei einer Temperatur zwischen 523 K und 773 K und einer Katalysatorbelastung von 1 g/h Edukt pro g Katalysator.

**Claims**

1. A process for the catalytic conversion of hydrocarbons and of their derivatives in petrochemical processes with the use of synthetic, crystalline aluminosilicates in shaped catalysts,
characterised in that
the catalytic conversion is carried out at pressures in the range of 0.1-15 MPa, at temperatures in the range of 523 K to 873 K and with quantities of catalyst of 0.5 to 10 g/h of educt per g of catalyst,
and that in the catalytic conversion
  - for the cracking of higher-boiling hydrocarbon fractions in a moving bed catalyst,
  - for the removal of n-paraffins and single-branched paraffins from hydrocarbon fractions,
  - of mixtures of $C_8$ aromatic compounds for the production of ortho- and/or para-xylenes,
  - for the alkylation of aromatic compounds with lower alkenes,
  - for the alkylation of aromatic compounds with alcohols,
  - for the isomerisation of lower n-paraffins to iso-paraffins,
  - for the production of aromatic compounds from lower hydrocarbons
and
  - of methanol for the production of liquid hydrocarbons or of lower alkanes and alkenes,
a synthetic crystalline aluminosilicate with the chemical composition
$$O\text{-}3M_2O : Al_2O_3 : 15\text{-}40\ SiO_2 : O\text{-}40\ H_2O,$$
wherein M is an alkali cation, is used as a catalyst or as a catalyst component, which has
  - X-ray reflections with at least the d-values (Å)

| | | | |
|---|---|---|---|
| 11.2 | ± | 0.3 | strong |
| 10.2 | ± | 0.3 | strong |
| 9.8 | ± | 0.2 | weak |
| 3.85 | ± | 0.1 | very strong |
| 3.83 | ± | 0.1 | strong |
| 3.75 | ± | 0.1 | strong |
| 3.73 | ± | 0.1 | strong |
| 3.60 | ± | 0.1 | weak |
| 3.06 | ± | 0.05 | weak |
| 3.00 | ± | 0.05 | weak |
| 2.01 | ± | 0.02 | weak |
| 1.99 | ± | 0.02 | weak |

- an Si/Al molar ratio at the surface of the crystallite greater than or equal to that within the crystallite, and

- absorption bands in the 29-silicon-solid state-MAS-nuclear resonance spectrum at around -100, -106, -112 and -116 ppm by reference to tetramethylsilane as standard.

2. A-process according to claim 1,
characterised in that
the catalytic cracking of higher-boiling hydrocarbons such as vacuum distillates and hydrogenated gas oils is carried out at a temperature between 723 K and 773 K and with a quantity of catalyst of 10 g/h educt per g of catalyst.

3. A process according to claim 1,
characterised in that
the catalytic conversion for the removal of n-paraffins and single-branched paraffins from hydrocarbon fractions is carried out at pressures in the range 1.0 to 1.5 MPa and at temperatures in the range of 523 K to 723 K.

4. A process according to claims 1 and 3,
characterised in that
paraffins are removed from a gas oil at a temperature of between 663 K and 723 K and with a quantity of catalyst of 2 g/h of gas oil per g of catalyst.

5. A process according to claim 1,
characterised in that
the catalytic conversion of mixtures of $C_8$ aromatic compounds for the production of ortho- and/or para-xylenes is carried out at pressures in the range of 0.5 to 5.0 MPa and at temperatures in the range of 523 K to 773 K.

6. A process according to claims 1 and 5,
characterised in that
for the isomerisation of xylene a mixture of $C_8$ aromatic compounds is catalytically converted at a temperature of between 623 K and 723 K and with a quantity of catalyst of 2 g/h of educt per g of catalyst.

7. A process according to claim 1,
characterised in that
the catalytic conversion for the alkylation of aromatic compounds with lower alkenes is carried out at pressures in the range of 1.0 to 5.0 MPa and at temperatures in the range of 623 K to 773 K.

8. A process according to claims 1 and 7,
characterised in that

16

benzene is reacted with ethylene to form ethylbenzene.

9. A process according to claims 1, 7 and 8,
characterised in that
a mixture of benzene and ethylene in a proportion by mass of benzene : ethylene = 1 : 2.6 is converted to ethylbenzene at a temperature of between 673 K and 693 K with a quantity of catalyst of 6.5 g/h educt per g of catalyst.

10. A process according to claims 1 and 7,
characterised in that
benzene is reacted with ethylene to form cumene.

11. A process according to claim 1,
characterised in that
the catalytic conversion for the alkylation of aromatic compounds with alcohols is carried out at pressures in the range of 0.1 to 0.5 MPa and at temperatures in the range of 523 K to 773 K.

12. A process according to claims 1 and 11,
characterised in that
toluene is reacted with methanol to form xylene.

13. A process according to claims 1, 11 and 12,
characterised in that
for the alkylation of toluene with methanol a mixture in a molar ratio of toluene : methanol = 2 : 1 is converted at temperature between 573 K and 673 K with a quantity of catalyst of 4 g/h educt per g of catalyst.

14. A process according to claim 1,
characterised in that
the catalytic conversion for the isomerisation of lower n-paraffins to iso-paraffins is carried out at pressures in the range of 0.5 to 5 MPa and at temperatures in the range of 473 K to 773 K.

15. A process according to claims 1 and 14,
characterised in that
the n-paraffins are isomerised at a temperature of between 523 K and 623 K and with a quantity of catalyst of 1 g/h educt per g of catalyst.

16. A process according to claim 1,
characterised in that
the catalytic conversion for the production of aromatic compounds from lower hydrocarbons is carried out at pressures of 0.1 to 5.0 MPa and at temperatures in the range of 773 K to 873 K.

17. A process according to claims 1 and 16,
characterised in that
for the production of aromatic compounds, nydrocarbons of low molecular weight are converted at a temperature of between 773 K and 873 K and with a quantity of catalyst of 1 g/h educt per g of catalyst.

18. A process according to claim 1,
characterised in that
the catalytic conversion of methanol for the production of liquid hydrocarbons or of lower alkanes and alkenes is carried out at pressures in the range of 0.1 to 5.0 MPa and at temperatures in the range of 523 K to 823 K.

19. A process according to claims 1 and 18,
characterised in that
the methanol is catalytically converted to liquid hydrocarbons of from $C_4$ to $C_{12}$ or to lower alkenes $< C_6$ at a temperature of between 523 K and 773 K and with a quantity of catalyst of 1 g/h educt per g of catalyst.

**Revendications**

1. Procédé pour la conversion catalytique d'hydrocarbures et de leurs dérivés dans des opérations pétroléochimiques avec utilisation d'aluminosilicates cristallins synthétiques dans des catalyseurs façonnés, caractérisé en ce que :
   la réaction catalytique est effectuée à des pressions dans l'intervalle de 0,1 à 15 MPa à des températures dans l'intervalle de 523 K à 873 K et avec des charges du catalyseur de 0,5 à 10 g/h d'éduct par g de catalyseur, en ce que, dans la conversion catalytique
   - pour craquage de fractions d'hydrocarbures à haut point d'ébullition sur un lit de catalyseur mobile,
   - pour élimination des n-paraffines et des paraffines monoramifiées de fractions d'hydrocarbures,
   - de mélanges d'hydrocarbures aromatiques en C 8 pour obtention d'ortho- et/ou de para-xylène,
   - pour alkylation d'hydrocarbures aromatiques à l'aide d'alcènes inférieurs,
   - pour alkylation d'hydrocarbures aromatiques à l'aide d'alcools,
   - pour isomérisation de n-paraffines inférieures en iso-paraffines,
   - pour obtention d'hydrocarbures aromatiques à partir d'hydrocarbures inférieurs, et
   - du méthanol pour obtention d'hydrocarbures liquides ou d'alcanes et d'alcènes inférieurs,
   on utilise en tant que catalyseur ou en tant que composant de catalyseur un aluminosilicate cristallin synthétique de composition chimique
   0 à 3 $M_2O$ : $Al_2O_3$ : 15 à 40 $SiO_2$ : 0 à 40 $H_2O$,
   dans laquelle M représente un cation métallique, et qui présente
   - au moins les réflexions de rayons X correspondant aux valeurs de d suivantes (Å) :

   | | | |
   |---|---|---|
   | 11,2 | +/- 0,3 | intense |
   | 10,2 | +/- 0,3 | intense |
   | 9,8 | +/- 0,2 | faible |
   | 3,85 | +/- 0,1 | très intense |
   | 3,83 | +/- 0,1 | intense |
   | 3,75 | +/- 0,1 | intense |
   | 3,73 | +/- 0,1 | intense |
   | 3,60 | +/- 0,1 | faible |
   | 3,06 | +/- 0,05 | faible |
   | 3,00 | +/- 0,05 | faible |
   | 2,01 | +/- 0,02 | faible |
   | 1,99 | +/- 0,02 | faible, |

   - un rapport des nombres de moles de Si/Al à la surface des cristallites qui est supérieur ou égal au rapport correspondant à l'intérieur des cristallites,
     et
   - des bandes d'absorption du spectre de résonance nucléaire MAS de corps solide de 29-silicium, étalon : tétraméthylsilane, à environ -100, -106, -112 et -116 ppm.

2. Procédé selon revendication 1, caractérisé en ce que le craquage catalytique d'hydrocarbures à haut point d'ébulLition tels que des distillats sous vide et des gazoles hydrogénés est réalisé à une température de 723 K à 773 K et à une charge de catalyseur de 10 g/h d'éduct par g de catalyseur.

3. Procédé selon revendication 1, caractérisé en ce que la conversion catalytique pour élimination des n-paraffines et des paraffines monoramifiées de fractions d'hydrocarbures est réalisée à des pressions dans l'intervalle de 1,0 à 1,5 MPa et à des températures de 523 K à 723 K.

4. Procédé selon les revendications 1 et 3,
   caractérisé en ce que
   on déparaffine un gazole à une température de 623 K à 723 K et avec une charge de catalyseur de 2 g/h de gazole par g de catalyseur.

5. Procédé selon la revendication 1,
caractérisé en ce que
la conversion catalytique de mélanges d'hydrocarbures en C 8 pour obtention d'ortho- et/ou de para-xylène est réali.sée à des pressions dans l'intervalle de 0,5 à 5,0 MPa et des températures dans l'intervalle de 523 K à 773 K.

6. Procédé selon les revendications 1 et 5,
caractérisé en ce que,
pour l'isomérisation du xylène, on soumet à conversion catalytique un mélange d'hydrocarbures aromatiques en C 8 à une température de 623 K à 723 K et avec une charge de catalyseur de 2 g/h d'éduct par g de catalyseur.

7. Procédé selon revendication 1,
caractérisé en ce que
la conversion catalytique pour alkylation d'hydrocarbures aromatiques à l'aide d'alcènes inférieurs est effectuée à des pressions dans l'intervalle de 1,0 à 5,0 MPa et des températures de 623 K à 773 K.

8. Procédé selon les revendications 1 et 7,
caractérisé en ce que
on convertit le benzène, par réaction avec l'éthylène, en éthylbenzène.

9. Procédé selon les revendications 1, 7 et 8,
caractérisé en ce que
on soumet à conversion catalytique en éthylbenzène un mélange de benzène et d'éthylène à un rapport en poids benzène/éthylène de 1:2,6 à une température de 673 K à 693 K et avec une charge de catalyseur de 6,5 g/h d'éduct par g de catalyseur.

10. Procédé selon les revendications 1 et 7,
caractérisé en ce que
on convertit le benzène, par réaction avec le propylène, en cumène.

11. Procédé selon la revendication 1,
caractérisé en ce que
la conversion catalytique pour alkylation des hydrocarbures aromatiques à l'aide d'alcools est réalisée à des pressions dans l'intervalle de 0,1 à 0,5 MPa et à des températures dans l'intervalle de 523 K à 773 K.

12. Procédé selon les revendications 1 et 11,
caractérisé en ce que
on convertit le toluène en xylène par réaction avec le méthanol.

13. Procédé selon les revendications 1, 11 et 12,
caractérisé en ce que
pour l'alkylation du toluène à l'aide du méthanol on convertit un mélange toluène/méthanol au rapport molaire de 2:1 à une température de 573 K à 673 K et avec une charge de catalyseur de 4 g/h d'éduct par g de catalyseur.

14. Procédé selon revendication 1,
caractérisé en ce que
la conversion catalytique pour l'isomérisation des n-paraffines inférieures en isoparaffines est exécutée à des pressions dans l'intervalle de 0,5 à 5 MPa et à des températures dans l'intervalle de 473 K à 773 K.

15. Procédé selon les revendications 1 et 14,
caractérisé en ce que
les n-paraffines sont isomérisées à une température de 523 K à 623 K et avec une charge de catalyseur de 1 g/h d'éduct par g de catalyseur.

**16.** Procédé selon la revendication 1,
caractérisé en ce que
la conversion catalytique pour obtention d'hydrocarbures aromatiques à partir d'hydrocarbures inférieurs est réalisée à des pressions de 0,1 à 5,0 MPa et à des températures dans l'intervalle de 773 K à 873 K.

**17.** Procédé selon les revendications 1 et 16,
caractérisé en ce que
pour la préparation d'hydrocarbures aromatiques, on soumet des hydrocarbures à bas poids moléculaire à conversion catalytique à une température de 773 K à 873 K avec une charge de catalyseur de 1 g/h d'éduct par g de catalyseur.

**18.** Procédé selon revendication 1,
caractérisé en ce que
la conversion catalytique du méthanol pour obtention d'hydrocarbures liquides ou d'alcanes et d'alcènes inférieurs est réalisée à des pressions dans l'intervalle de 0,1 à 5,0 MPa et à des températures dans l'intervalle de 523 K à 823 K.

**19.** Procédé selon revendications 1 et 18,
caractérisé en ce que
on soumet le méthanol à conversion catalytique en hydrocarbures liquides de C 4 à C 12 ou en alcènes inférieurs < C 6 à une température de 523 K à 773 K et avec une charge de catalyseur de 1 g/h d'éduct par g de catalyseur.

Abb. 1: Alumosilikat Typ A

29Si

Si(3Si1Al)

Si(4Si0Al)

Si(2Si2Al)

-90   -100   -110   -120
PPM

Abb. 2: Alumosilikat Typ B

$^{29}$Si

Si(4Si0Al)

Si(3Si1Al)

Si(2Si2Al)

-90      -100      -110      -120
PPM

Abb. 3: Alumosilikat Typ C

$^{29}$Si

Si(4SiOAl)

Si(3Si1Al)

C

-80  -100  -120  -140

δ/ppm

Silizium

Aluminium

Abb. 4: Aluminiumverteilung über den
Kristallquerschnitt bei dem
Alumosilikat vom Typ A

Aluminium
Silizium

Abb. 5: Aluminiumverteilung über den
Kristallquerschnitt bei dem
Alumosilikat vom Typ B

Aluminium

Silizium

Abb. 6: Aluminiumverteilung über den
Kristallquerschnitt bei dem
herkömmlichen Vergleichsalumosilikat
vom Typ C